(19)

Europäisches
Patentamt
European
Patent Office
Office européen
des brevets

(11) **EP 1 927 857 A1**

(12) **EUROPEAN PATENT APPLICATION**
published in accordance with Art. 153(4) EPC

(43) Date of publication:
**04.06.2008 Bulletin 2008/23**

(21) Application number: **06797055.8**

(22) Date of filing: **30.08.2006**

(51) Int Cl.:
*G01N 33/00* (2006.01)    *G01N 33/22* (2006.01)
*G01N 24/08* (2006.01)    *G01N 21/35* (2006.01)
*G01N 27/62* (2006.01)

(86) International application number:
**PCT/JP2006/317082**

(87) International publication number:
**WO 2007/034663 (29.03.2007 Gazette 2007/13)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **21.09.2005 JP 2005274690**

(71) Applicant: **Idemitsu Kosan Co., Ltd.
Chiyoda-ku
Tokyo 100-8321 (JP)**

(72) Inventor: **TASHIRO, Hironori
Chiba, 299-0193 (JP)**

(74) Representative: **Vossius & Partner
Siebertstrasse 4
81675 München (DE)**

(54) **METHOD OF DETERMINING CYCLIC CARBON CONTENT IN SAMPLE AND METHOD OF DETERMINING CONTENT OF CYCLIC CARBON MATERIAL IN SAMPLE**

(57)    A method for determining a cyclic carbon content in a sample including burning a sample to generate carbon dioxide gas and measuring a total $^{14}$C amount in the sample from the carbon dioxide gas; burning the sample to confirm presence or absence of ash; when ash is present, treating the sample with an acid to generate carbon dioxide gas and measuring a $^{14}$C amount from the carbon dioxide gas; correcting the total $^{14}$C amount in the sample with the $^{14}$C amount measured by the acid treatment; and calculating a cyclic carbon content in the sample from the corrected total $^{14}$C amount in the sample.

FIG. 1

STEP A

MEASUREMENT OF TOTAL $^{14}$C
CONCENTRATION (pMC: B%) OF
SAMPLE BY COMBUSTION TREATMENT

STEP B

CONFIRMATION OF
PRESENCE OR ABSENCE
OF ASH IN SAMPLE
ABSENT → CYCLIC CARBON CONTENT (%)
OF SAMPLE = (B/110) × 100

STEP C    PRESENT

MEASUREMENT OF $^{14}$C
CONCENTRATION (pMC: D%) OF
SAMPLE BY ACID TREATMENT

STEP D

CORRECTION(1)

CONFIRMATION OF
PRESENCE OR ABSENCE
OF CARBONATE ESTER
IN SAMPLE
ABSENT → ASSUME D AS $^{14}$C
CONCENTRATION
(pMC: E%) OF
CARBONATE SALT → CALCULATION OF $^{14}$C
CONCENTRATION (pMC: F%)
OF SAMPLE EXCLUDING
CARBONATE SALT → CYCLIC CARBON CONTENT
(%) OF SAMPLE EXCLUDING
CARBONATE SALT =
(F/110) × 100

PRESENT
CORRECTION(2)

ASSUME D AS $^{14}$C
CONCENTRATION (pMC:
H%) OF CARBONATE SALT
AND CARBONATE ESTER → CALCULATION OF
$^{14}$C CONCENTRATION
(pMC: E'%) OF
CARBONATE SALT → CALCULATION OF $^{14}$C
CONCENTRATION (pMC: F'%)
OF SAMPLE EXCLUDING
CARBONATE SALT → CYCLIC CARBON CONTENT
(%) OF SAMPLE EXCLUDING
CARBONATE SALT =
(F'/110) × 100

**Description**

TECHNICAL FIELD

The present invention relates to a method for determining the cyclic carbon content in a sample and a method for determining the content of cyclic carbon materials in a sample.

BACKGROUND ART

In recent years, the concept of the biomass degree (cyclic carbon content) has spread on account of the trend of regulating emission of carbon dioxide into the atmosphere. The biomass degree is a measure for evaluating a substance in view of suppressing an increase of carbon dioxide in the atmosphere. Specifically, carbon contained in a sample is grouped into "cyclic carbon" in the food chain cycle in the biosphere and "buried carbon" of fossil fuel origin such as petroleum, coal, and the like. A substance with a high cyclic carbon content is regarded as a material with a high biomass degree. It is thought that a material with a high biomass degree does not contribute much to an increase of carbon dioxide, since such a material is only returned to the atmospheric recycle system in the case of conversion into carbon dioxide by burning.
Specifically, a substance originating from animals and plants such as polylactic acid is regarded as having a 100% biomass degree. Such a substance is not involved in emission of carbon dioxide generated from buried carbon. On the other hand, a polyolefin resin, a polyester resin, and the like of which the raw material is a fossil fuel is regarded to have a biomass degree of 0%.
A polymer, such as polylactic acid, which is a biomass component is generally brittle. Thus, when using the polymer as a material, the polymer is often used as a composition containing a resin component or inorganic additive of fossil fuel origin to impart plasticity and rigidity. Since various biomass components which do not always have a high biomass degree are added, the compositions actually used have different biomass degrees.
For this reason, development of a simple and convenient method for measuring the amount of materials using cyclic carbon (biomass components) in a sample (biomass material) has been desired.
As a technology using a $^{14}$C concentration, Patent Document 1 discloses a method for measuring a rate of biodegradation of a non-natural organic compound by measuring the concentration of $^{14}$C in carbon dioxide produced by biodegradation in the presence of a biodegradation culture medium of the non-natural organic compound by accelerator mass spectrometry and determining the rate of biodegradation from the reduction rate of the $^{14}$C concentration from the $^{14}$C concentration of modern carbon. Patent Document 2 also discloses, in the biodegradation of a non-natural organic compound in the presence of a biodegrading culture medium, a method of measuring the concentration of a radioactive carbon isotope $^{14}$C in the biodegrading culture medium and determining the biodegradation ratio of the non-natural organic compound from the difference between the measured concentration of $^{14}$C and the concentration of $^{14}$C in modern carbon.
However, the techniques disclosed by Patent Document 1 and Patent Document 2 relate to a method of measuring the rate of biodegradation of non-natural organic compounds. No specific method of measuring the content of cyclic carbonaceous material has been known.
[Patent Document 1] JP-A-2003-185634
[Patent Document 2] JP-A-2004-198239
The invention has been achieved in view of this situation. An obj ect of the invention is to provide a method for determining a more accurate content of cyclic carbon in a sample by correcting the effect of additives contained in the sample and further provide a simple and convenient method for determining the content of a material using cyclic carbon (biomass components) in a sample.

DISCLOSURE OF THE INVENTION

The invention provides the following methods for determining the cyclic carbon content in a sample and a method for determining the content of cyclic carbon material in a sample.

1. A method for determining a cyclic carbon content in a sample comprising:

burning a sample to generate carbon dioxide gas and measuring a total $^{14}$C amount in the sample from the carbon dioxide gas;
burning the sample to confirm presence or absence of ash;
when ash is present, treating the sample with an acid to generate carbon dioxide gas and measuring a $^{14}$C amount from the carbon dioxide gas;

correcting the total $^{14}$C amount in the sample with the $^{14}$C amount measured by the acid treatment; and calculating a cyclic carbon content in the sample from the corrected total $^{14}$C amount in the sample.

2. The method according to 1, wherein the $^{14}$C amount measured by the acid treatment is taken as a $^{14}$C amount originating from carbonate salt contained in the sample; and a cyclic carbon content of the portion excluding the carbonate salt of the sample and/or a cyclic carbon content of the carbonate salt portion are/is calculated.

3. The method according to 1, wherein presence or absence of carbonate ester in the sample is measured; when carbonate ester is present, the $^{14}$C amount measured by the acid treatment is taken as a $^{14}$C amount originating from carbonate salt and carbonate ester; a $^{14}$C amount originating from the carbonate salt is calculated from the $^{14}$C amount measured by the acid treatment; and a cyclic carbon content of the portion excluding the carbonate salt of the sample and/or a cyclic carbon content of the carbonate salt portion are/is calculated.

4. The method according to 3, wherein the presence or absence of carbonate ester is confirmed by $^{13}$C NMR analysis and IR absorption analysis.

5. The method according to 3 or 4, wherein an amount of the carbonate ester is determined by an integrated value obtained by $^{13}$C NMR analysis.

6. The method according to 5, wherein an amount of carbon dioxide gas originating from the carbonate ester in the carbon dioxide gas generated by the acid treatment is calculated, based on the amount of the carbonate ester in the sample; and the cyclic carbon content of the carbonate salt portion is determined by subtracting the amount of carbon dioxide gas originating from the carbonate ester from the amount of carbon dioxide gas generated by the acid treatment.

7. The method according to any one of 1 to 6, wherein the cyclic carbon content in the sample is determined by calculating a cyclic carbon content of the portion excluding carbonate salt of the sample and a cyclic carbon content of the carbonate salt portion, and adding the resultant cyclic carbon contents.

8. A method for determining a content of a cyclic carbon material in a sample comprising:

determining a cyclic carbon content in a sample according to the method recited in any one of 1 to 7; and calculating a content (mass%) of a cyclic carbon material from the determined cyclic carbon content in the sample, a total carbon amount of the sample, and a carbon content of each component contained in the sample.

According to the method for determining the cyclic carbon content in a sample of the invention, the cyclic carbon content of components of the sample, specifically, the cyclic carbon contents of resin and carbonate salt can be determined.

In addition, according to the method for determining content of a material containing cyclic carbon in a sample, it is possible to measure the mass percent (wt%) of a biomass component in the sample. Furthermore, it is possible to measure the mass fraction (wt%) of eggshell and the like, which are a biomass component, among carbonate salts in the biomass component.

BRIEF DESCRIPTION OF THE DRAWINGS

Figure 1 is a flow chart of the method of measuring the cycle carbon content in a sample, excluding carbonate salt, of the invention.
Figure 2 is a schematic drawing showing an example of the device converting a solid sample into carbon dioxide.

BEST MODE FOR CARRYING OUT THE INVENTION

The method for determining the cyclic carbon content of the invention comprises the following steps:

(A) the step of measuring the total amount of $^{14}$C in a sample from carbon dioxide gas generated by burning the sample,
(B) the step of identifying the presence or absence of ash when the sample is burnt,
(C) the step of measuring the amount of $^{14}$C in carbon dioxide gas generated by treating the sample with an acid, when ash is confirmed to be present, and
(D) the step of correcting the total $^{14}$C amount in the sample with the $^{14}$C amount determined by the acid treatment, and calculating the cyclic carbon content in the sample from the corrected total $^{14}$C amount in the sample.
Each step is described below.

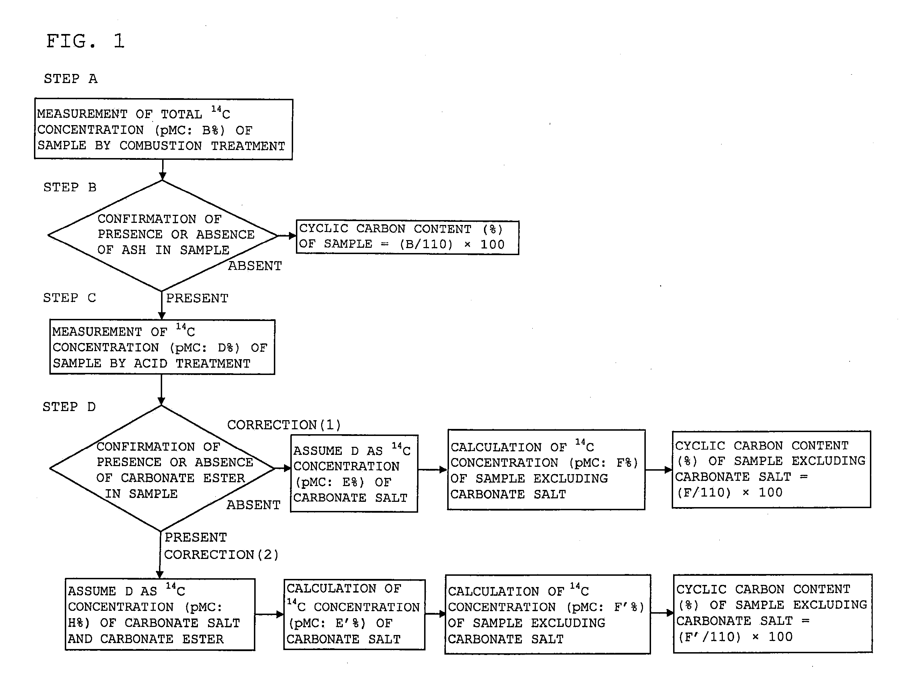

A flow chart of the method of measuring the cyclic carbon content in a sample according to the invention is shown in Figure 1.

Step (A)

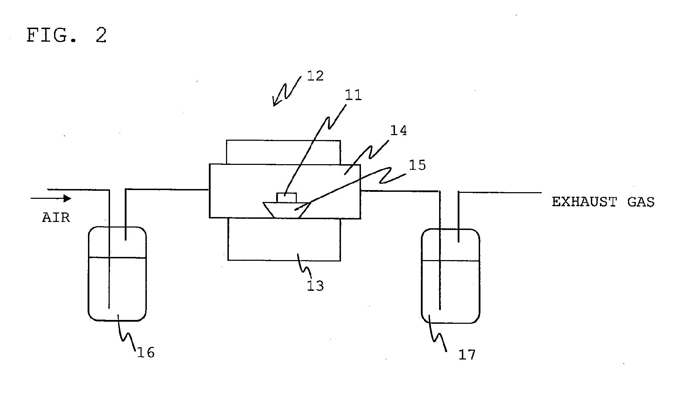

In the method of the invention, the sample which is an object to be measured is first burned to convert all the carbon element contained in the sample into carbon dioxide. The conversion into carbon dioxide can be carried out using a device for converting a solid sample into carbon dioxide as shown in Figure 2, for example.
In Figure 2, air for burning a sample 11 is fed to a combustor 12 in which the sample is burned. The combustor 12 has a heating means 13, a quartz tube 14 in which the sample 11 is placed, and a ceramic combustion boat 15. Carbon dioxide is previously removed from the air which is sent to the combustor 12. Carbon dioxide can be removed by, for example, passing the air through a trap 16 of a basic substance such as an aqueous solution of sodium hydroxide.
Combustion of a sample is preferably carried out by heating the sample 11 at 900 to 1,000˚C in the combustor 12, while introducing air free from carbon dioxide. Almost all carbon in the sample can be converted into carbon dioxide by burning the sample at temperatures in this range.
The combustion gas containing carbon dioxide generated from the sample is sent to a trap 17 containing a basic substance such as an aqueous solution of sodium hydroxide, by which the carbon dioxide is collected.
The total $^{14}$C concentration of the sample is measured from the collected carbon dioxide. The total $^{14}$C concentration can be measured using an accelerator mass spectroscopy meter, for example.
As an international notation of $^{14}$C concentration, a value calculated on the basis of the $^{14}$C concentration in the cyclic carbon in the year 1950 as 100% is referred to as pMC (percent Modern Carbon). The concentration of radiocarbon isotope $^{14}$C in carbon dioxide in the atmosphere has changed from 1950, and the $^{14}$C carbon concentration by 2005 was in a range of 110 to 111%.
In this step, it is a preferable to measure the total carbon amount (TOC) in a sample. It is possible to calculate the content of a biomass component (mass %) in a sample by measuring the total carbon amount.
The total carbon amount can be determined by measuring the carbon dioxide concentration absorbed by a basic compound in a trap by elementary analysis and multiplying the mass of the basic compound in the trap by the carbon dioxide concentration.

Step (B)

In this step, the presence or absence of ash when the sample is burned is identified. In order to save measuring time, it is preferable to measure the ash produced by burning in the step (A).
The presence of ash in this step indicates that the sample contains an inorganic additive such as a filler. In such a case, it is necessary to correct the effect of the inorganic additive when measuring the cyclic carbon content.
When no ash is detected in this step, indicating that the sample does not contain an inorganic additive, the cyclic carbon content can be calculated based on the total $^{14}$C concentration measured in the step (A). Specifically, the cyclic carbon content in the sample can be calculated according to the following formula (1).

$$\text{Cyclic carbon content of sample (\%) =}$$

$$\text{(Total } ^{14}\text{C concentration)/110} \qquad \text{(1)}$$

wherein the $^{14}$C concentration in the cyclic carbon in 2005 is assumed to be 110.

Step (C)

When ash is confirmed to be present, the $^{14}$C concentration is measured from the carbon dioxide gas generated by treating the sample with an acid. The $^{14}$C concentration measured here is the concentration of $^{14}$C originating mainly from salt of carbonic acid (carbonate salt) and/or ester of carbonic acid (carbonate ester) contained in the sample. Therefore, the cyclic carbon content in a sample excluding the effect of carbonate salt and the like can be calculated by correcting the total $^{14}$C concentration of the sample measured in the step (A) with the $^{14}$C concentration measured

in this step.

As an example of the method for treating the sample, a method of charging a flask with a prescribed amount of the sample, tightly sealing the flask, replacing the inside of the flask with nitrogen or the like to remove carbon dioxide, and adding an acid to collect generated carbon dioxide can be given. Concentrated hydrochloric acid, phosphoric acid, and the like can be used as the acid for treating the sample.

The $^{14}$C concentration of the sample is measured from the collected carbon dioxide. The same as in the step (A), the $^{14}$C concentration can be measured using an accelerator mass spectroscopy meter.

Similarly to the step (A), it is preferable to measure the total carbon amount (TOC) in the sample also in this step.

Step (D)

In this step, the total $^{14}$C amount in the sample is corrected with the $^{14}$C amount determined by the acid treatment, and the cyclic carbon content in the sample is calculated from the corrected total $^{14}$C amount in the sample. As the method of correction, the following method (1) and method (2) can be given.

(1) A correcting method by assuming the amount of $^{14}$C measured by the acid treatment to be the value of the carbon originating from carbonate salt

In many cases, the sample to measure the cyclic carbon content is considered to be a composition comprising biomass components and additives (non-biomass components) which are added as a filler or a reinforcing material. Among the additives, the main components generating carbon dioxide gas by the acid treatment are thought to be carbonate salt represented by calcium carbonate and carbonate ester such as polycarbonate. Therefore, in the case in which carbonate ester is not added or added only in a very small amount, or in the case in which it is judged that the degree of acid decomposition is small and carbon dioxide originating from carbonate ester is generated in a small amount, the cyclic carbon content of the portion excluding carbonate salt in the sample and/or the cyclic carbon content of the carbonate salt portion in the sample can be calculated on the basis of the above assumption.

Specifically, the total $^{14}$C concentration (pMC: B%) of the samples, the total carbon amount (P g) of the samples, the amount of $^{14}$C measured by the acid treatment (pMC: D%), the total carbon amount (Q g) measured by the acid treatment, and the $^{14}$C concentration (pMC: F%) of the sample excluding the carbonate salt, measured in each of the steps mentioned above, have the relationship of the following formula (2).

$$(P - Q) \times F + Q \times D = P \times B \qquad (2)$$

Therefore, the $^{14}$C concentration (pMC: F%) of the sample excluding carbonate salt is shown by the following formula (3).

$$F\ (\%) = (P \times B - Q \times D)/(P - Q) \qquad (3)$$

From the $^{14}$C concentration (pMC: F%) of the portion excluding carbonate salt in the sample, the cyclic carbon content of this portion can be calculated by the following formula (4).

$$\text{Corrected cyclic carbon content of sample (\%)} =$$
$$(F \times 100)/110 \qquad (4)$$

(2) A correcting method by assuming the amount of $^{14}$C measured by the acid treatment to be the value of the carbon originating from carbonate salt and ester.

In the case in which a large amount of carbonate ester is added or in the case in which a large amount of carbon dioxide is generated due to abundant acid decomposition of the carbonate ester, the cyclic carbon content of the portion excluding carbonate salt in a sample can be more accurately calculated than (1) on the basis of the above assumption.

In this method, the presence or absence of carbonate ester is first identified.

The presence or absence of carbonate ester can be identified by $^{13}C$ NMR and IR absorption, for example. Specifically, absorbance between 150 ppm to 180 ppm in a spectrum obtained by $^{13}C$ NMR analysis (solid $^{13}C$ NMR) is measured. Further, absorbance between 1,600 to 1,800 $cm^{-1}$ in a spectrum obtained by IR analysis is measured. When the absorption is confirmed in both measurements, carbonate ester is judge to be present.

The amount of the carbonate ester in a sample can be determined by preparing a standard compound calibration curve and applying the NMR integrated value obtained by the above-mentioned measurement.

The amount of $^{14}C$ originating from carbonate salt is calculated from the amount of $^{14}C$ measured by the acid treatment.

Specifically, the $^{14}C$ concentration (pMC: E'%) of carbonate salt can be calculated from the $^{14}C$ amount (pMC: D%) measured by the acid treatment, the total carbon amount measured by the acid treatment [R = S + T(g)], the total carbon amount originating from carbonate salt (S g), and the total carbon amount originating from carbonate ester (T g) according to the following formula (5).

$$E' \ (\%) \ = \ D \ (S \ + \ T)/S \qquad (5)$$

The $^{14}C$ concentration (pMC: F'%) of the sample excluding carbonate salt can be calculated from the total $^{14}C$ concentration (pMC: B%) of the sample measured in the step (A), the total carbon amount of the sample (Ug), the $^{14}C$ concentration (pMC: E'%) of carbonate salt, and the total carbon amount originating from carbonate salt (S g) according to the following formula (6).

$$F' \ (\%) \ = \ (U \ \times \ B \ - \ E' \ \times \ S)/(U \ - \ S) \qquad (6)$$

From the $^{14}C$ concentration (pMC: F'%) of the portion excluding carbonate salt in the sample, the cyclic carbon content of this portion can be calculated by the following formula (7).

$$\text{Cyclic carbon content of the portion excluding carbonate salt}$$

$$\text{in the sample (\%)} \ = \ (F' \ \times \ 100)/110 \qquad (7)$$

The total carbon amount originating from carbonate salt (S g) and the total carbon amount originating from carbonate ester (T g) can be estimated from the total carbon amount measured by the acid treatment [R = S + T(g)] and the amount of carbonate ester in the sample.

For example, using standard samples containing polylactic acid as a polymer originating from animals and plants, polycarbonate (PC) as carbonate ester, and calcium carbonate ($CaCO_3$) as carbonate salt, in which the ratio of these components is changed, the amount of carbon dioxide originating from PC is determined from the amount of carbon dioxide generated when the standard samples are treated with an acid and the calculated amounts of carbon dioxide originating from calcium carbonate.

The amount of change K (= amount of carbon dioxide originating from PC/amount of PC) can be determined by measuring the amount of carbon dioxide originating from PC, while changing the amount of PC and maintaining the amount of calcium carbonate constant, plotting the amount of PC along the horizontal axis and the amount of carbon dioxide originating from PC along the vertical axis, and determining the inclination (the amount of change K) of the resulting line in the chart.

The amount of change β (= amount of carbon dioxide originating from PC/amount of PC·amount of calcium carbonate) can be determined by obtaining several points of the amount of change K in the same way for samples with different amounts of calcium carbonate, plotting the amount of calcium carbonate along the horizontal axis and the amount change K along the vertical axis, and determining the inclination (the amount of change β of the resulting line in the chart.

The amount of carbon dioxide originating from carbonate ester can be calculated by applying the amount of change β, the amount of PC, and the amount of calcium carbonate to the following formula (8).

$$\text{Amount of carbon dioxide of carbonate ester (wt\%) =}$$

$$\beta \times \text{amount of PC (wt\%)} \times$$

$$\text{amount of calcium carbonate (wt\%)} \qquad (8)$$

Since the rate of acid decomposition of PC in the standard samples is small, the total carbon amount measured by acid treatment [R = S + T (g)] is assumed to be the amount originating from decomposition of calcium carbonate. Based on this assumption, the amount (wt%) of calcium carbonate is calculated from the total carbon amount according to the following formula (9).

$$\text{Amount of calcium carbonate (wt\%) =}$$

$$R \times 100 \times M_{CaCO3}/Mc \qquad (9)$$

wherein $M_{CaCO3}$ is the molecular weight of calcium carbonate (100) and Mc is the elemental weight of carbon (12).
The amount of carbon dioxide originating from carbonate ester (wt%) in the carbon dioxide generated in the acid treatment can be calculated by assigning the resulting amount of calcium carbonate to the formula (8). The total carbon amount originating from carbonate ester (Tg = amount of carbon dioxide originating from carbonate ester × 12/44) can be determined therefrom, and then, the amount of total carbon originating from carbonate salt (Sg = R - T) can be calculated.
As explained above, according to the invention, for the cyclic carbon content (pMC: %) of a sample, the cyclic carbon content of a sample excluding carbonate salt, that is, the corrected cyclic carbon content, in which the effect of carbon originating from the carbonate salt has been corrected, can be measured.
In addition, since the $^{14}C$ concentration of the portion of carbonate salt in a sample [pMC: E' %, the amount of $^{14}C$ (pMC: D%) measured by acid treatment in the above assumption (1)] is determined in the course of measurement, the cyclic carbon content of the portion of carbonate salt can also be calculated.
For example, eggshell and seashell contain calcium carbonate mainly made of cyclic carbon (biomass carbonate salt) and limestone contains calcium carbonate mainly made of non-cyclic carbon (non-biomass carbonate salt). When the pMC of $^{14}C$ amount in the biomass carbonate salt in a sample is G%, the following formula (10) applies.

$$^{14}C \text{ concentration of carbonate salt =}$$

$$\text{Carbon amount of biomass carbonate salt (wt\%)} \times$$

$$\text{G/total carbon amount of carbonate salt (wt\%)} \qquad (10)$$

The amount of biomass carbonate salt (wt%) can be calculated by calculating the amount of carbon of biomass carbonate salt using this formula and dividing the resulting amount by the carbon content of the carbonate salt ($Mc/M_{CaCO3}$). In addition, in the case of a sample containing several types of biomass carbonate salts, the amount of each biomass carbonate salt in the sample can be calculated if the composition of the carbonate salts in the sample and each pMC are known.
According to the invention, the content (mass%) of a cyclic carbon substance (biomass component) in a sample can be calculated from the cyclic carbon content in the sample; the total carbon amount of the sample, or the total carbon content and total carbon amount generated by the acid treatment of the sample; determined by the method mentioned above, and the carbon content of each material contained in the sample.
Specifically, the mass% of the biomass component can be calculated by multiplying the cyclic carbon content (%) in the sample by the total carbon amount (g) of the sample to obtain the mass percent (wt%) of cyclic carbon in the sample, and by dividing the result by the carbon content of each material contained in the sample. The carbon content (wt%) of each material can be determined by elementary analysis. For example, the carbon content of polylactic acid is 50 wt%, and the carbon content of cellulose is 44.45 wt%.

In addition, even in the case in which two or more biomass components are contained in the sample, the content (mass %) of each component can be calculated if the carbon content (atomic %) and the mixed ratio of each material are known. The measurement methods of the invention will now be described more specifically by examples.

Examples

Example 1:

Treatment 1: Measurement of total amount of carbon and ash in sample by combustion

A sample was treated using a device for converting a solid sample into carbon dioxide shown in Figure 2 to collect carbon dioxide and to identify the presence or absence of ash.

1,000.0 mg of the sample was placed on a ceramic board and burned at 900°C to 1,000°C for six hours in a quartz tube of a combustor. Air not containing carbon dioxide was supplied to the quartz tube at a rate of 1 to 5 liter/min. Carbon dioxide was previously removed from the air by causing the air to flow through a trap of 1 liter of 2N NaOH aqueous solution. The combustion gas was caused to pass through a trap in which 1 liter of 2N NaOH aqueous solution was used to absorb the total amount of carbon dioxide generated from the sample. The mass of the NaOH aqueous solution which absorbed the carbon dioxide was taken as X (g).

After combustion, the weight of ash left on the ceramic board was measured to determine the presence or absence of ash.

A part (not more than 10 ml) of the NaOH aqueous solution which absorbed carbon dioxide was subjected to elementary analysis to determine the total carbon concentration (TOC) in the sample. The resulting concentration is indicated as A (wt ppm).

Based on the above experiment, the total carbon amount in 1,000 mg of the sample was determined by $X \times A \times 10^{-3}$ (mg) and the mass percent of carbon was determined by $10^{-4} \times X \times A$ (wt%).

Treatment 2: Measurement of total $^{14}C$ amount (pMC) in sample

Concentrated sulfuric acid was added dropwise to the NaOH aqueous solution which absorbed carbon dioxide in the Treatment 1 to remove carbon dioxide from the solution. In this instance, in order to prevent carbon dioxide in the atmosphere from entering, the atmosphere in the reactor was replaced with nitrogen by a nitrogen gas purge under reduced pressure, and the nitrogen gas purge was continued during the addition of the concentrated sulfuric acid.

The $^{14}C$ amount (total $^{14}C$ amount of the sample: pMC) of the collected carbon dioxide was measured using an accelerator mass spectroscopic meter ("Model 9SDH-2; 3.0 MV; "Tanden Pelletron" manufactured by U.S. NEC Corp.). The resulting value (pMC) is indicated as B%.

Treatment 3: Determination of presence or absence of carbonate ester in sample

50.0 mg of the sample was mixed with 50.0 mg of NaCl (diluent). The mixture was pulverized and subjected to IR absorption analysis (diffuse-reflection mode) to confirm the presence or absence of absorption between 1,600 to 1,800 cm$^{-1}$.

900.0 mg of the sample was mixed with 100 mg of 2-adamantanone (internal standard substance). The mixture was pulverized and subjected to $^{13}C$ NMR analysis (solid $^{13}C$ NMR) to confirm the presence of absence of absorption between 150 ppm and 180 ppm.

When the absorption was confirmed in both measurements, carbonate ester was judged to be present.

Treatment 4: Measurement of total carbon amount and $^{14}C$ amount (pMC) in sample by acid treatment

A flask was charged with 10.000 g of the sample. In this instance, in order to prevent carbon dioxide in the atmosphere from entering, the atmosphere in the flask was replaced with nitrogen by a nitrogen gas purge under reduced pressure, and the nitrogen gas purge was continued during the following dropwise addition of concentrated hydrochloric acid. 15 g of concentrated hydrochloric acid (34 to 36 wt%) was added dropwise to the sample to generate carbon dioxide from the sample.

The carbon dioxide was caused to pass through a trap in which 1 liter of 1N NaOH aqueous solution was used to absorb the total amount of carbon dioxide generated from the sample. The mass of the NaOH aqueous solution which absorbed the carbon dioxide was taken as Y (g).

A part (not more than 10 ml) of the NaOH aqueous solution which absorbed the carbon dioxide was subjected to elementary analysis to determine the total carbon concentration (TOC) in the sample. The resulting value is indicated as C (wt ppm).

Based on the above experiment, the total carbon amount in 10 g of the sample was determined by $Y \times C \times 10^{-3}$ (mg) and the carbon dioxide mass percent was determined by $Y \times C \times 10^{-6}$ (g) $\times (M_{CO2}/Mc)/10$ (g) $\times 100$ (wt%), that is, $(M_{CO2}/Mc) \times 10^{-5} \times Y \times C$ (wt%).

In the same manner as in Treatment 2, the $^{14}$C amount of the collected carbon dioxide ($^{14}$C amount in the sample when treated with an acid: pMC) was measured. The resulting value (pMC) is indicated as D%.

The values obtained in the above Treatments 1 to 4 were calculated for the following cases to calculate the cyclic carbon content (%) of the sample and the mass percent (wt%) of the biomass component contained in the sample.

(1) Case in which ash was not left in Treatment 1

Based on the Treatment 1, the total carbon amount in 1,000 mg of the sample was $X \times A \times 10^{-3}$ (mg) and based on the Treatment 2, the total $^{14}$C concentration of the sample was B%. Assuming that the $^{14}$C amount of a sample with a 100% cyclic carbon content is 110 (%), the cyclic carbon content H of the sample is shown by the following formula.

$$H\ (\%)\ =\ B/110\ \times\ 100$$

The mass percent I (wt%) of carbon originating from cyclic carbon (biomass component) can be calculated by the following formula.

$$I\ (wt\%)\ =\ X\ \times\ A\ \times\ 10^{-6}\ \times\ H$$

When the biomass component contained in the sample is polylactic acid, since the carbon content according to elementary analysis is 50 wt%, the mass percent of the polylactic acid contained in the sample is as follows.

$$Mass\ percent\ of\ polylactic\ acid\ (wt\%)\ =\ I/0.5$$

When the biomass component contained in the sample is cellulose, since the carbon content according to elementary analysis is 44.45 wt%, the amount of the mass percent of the cellulose contained in the sample is as follows.

$$Mass\ percent\ of\ cellulose\ (wt\%)\ =\ I/0.4445$$

(2) Case in which ash is judged to be present in Treatment 1 and carbonate ester is judged not to be present in sample in Treatment 3

All carbon dioxide generated in the acid treatment in Treatment 4 can be assumed to be originated from carbonate salt. Therefore, the total carbon amount originating from the carbonate salt in 10 g of the sample is $Y \times C \times 10^{-3}$ (mg) and the $^{14}$C concentration originating from the carbonate salt is D%.

Referring to the formula (2), the $^{14}$C concentration of the portion excluding carbonate salt of the sample (pMC: F%) can be determined from the total $^{14}$C concentration (pMC: B%) of the sample, the total carbon amount in 1 g of the sample $(X \times A \times 10^{-3}$ (mg)), the $^{14}$C concentration originating from carbonate salt (pMC: D%), and the total carbon amount originating from carbonate salt $(Y \times C \times 10^{-4}$ (mg) : per 1 g of sample) according to the following formula.

$$F(\%) = \frac{B \times X \times A \times 10^{-6} - D \times Y \times C \times 10^{-7}}{X \times A \times 10^{-6} - Y \times C \times 10^{-7}}$$

From the $^{14}$C concentration (pMC: F%) of the portion excluding carbonate salt of the sample, the cyclic carbon content H' (%) of this portion can be calculated by the following formula.

$$H' \; (\%) \; = \; (F \; \times \; 100)/110$$

The mass percent I' of carbon (wt%) originating from cyclic carbon (biomass component) can be calculated by the following formula.

$$I' \; (wt\%) \; = \; (X \; \times \; A \; \times \; 10^{-6} \; - \; Y \; \times \; C \; \times \; 10^{-7}) \; \times \; H'$$

In the same manner as in (1) mentioned above, the amount of each component in the sample is calculated from the carbon content of the biomass component.

(3) Case in which ash is judged to be present in Treatment 1 and carbonate ester is judged to be present in sample in Treatment 3

(a) Determination of amount of carbonate ester in a sample

The amount of carbonate ester in a sample can be determined by preparing a standard compound calibration curve by $^{13}$C NMR analysis (solid $^{13}$C NMR) and applying the NMR integrated value obtained by the above-mentioned measurement. Specifically, using polycarbonate (PC, carbon content: 75.57 wt%) as a conversion substance and 2-admantanone (ADO) as an internal standard substance, integrated values of the chemical shift resulting from C=O of PC (145 to 160 ppm) and the chemical shift resulting from C=O of ADO (210 to 250 ppm) were measured using samples with different ratios of PC and ADO (PC wt%/ADO wt%) to determine the integrated value of PC when the integrated value between 210 to 250 ppm is 100. A chart is prepared by plotting the integrated values of PC along the vertical axis and the ratios of PC to ADO along the horizontal axis to determine the inclination (percentage of change $\alpha$ = integrated value of PC/[PC wt%/ADO wt%]) Then, the amount of carbonate ester (as PC) contained in a sample can be calculated by the following formula.

$$\text{Amount of carbonate salt (PC wt\%)} =$$

$$\text{integrated value of PC/} \; \alpha \; \times \; (ADO \; wt\%)$$

(b) Amount of carbon dioxide originating from carbonate ester in carbon dioxide generated by acid treatment

The effect of carbonate ester on the amount of carbon dioxide generated by the acid treatment was estimated by the above-mentioned method. Specifically, using standard samples containing polylactic acid as a polymer originating from animals and plants, polycarbonate (PC) as carbonate ester, and calcium carbonate ($CaCO_3$) as carbonate salt, in which the ratio of these components is changed, the amount of carbon dioxide originating from the carbonate ester was determined from the amount of carbon dioxide generated when the standard sample was treated with an acid and the calculated amount of carbon dioxide originating from calcium carbonate.
The amount of change K (= amount of carbon dioxide originating from PC/amount of PC) can be determined by measuring the amount of carbon dioxide originating from PC, while changing the amount of PC and maintaining the amount of calcium carbonate constant, plotting the amount of PC along the horizontal axis and the amount of carbon dioxide originating from PC along the vertical axis, and determining the inclination (the amount of change K) of the resulting line in the chart.

The amount of change β (= amount of carbon dioxide originating from PC/amount of PC·amount of calcium carbonate) can be determined by obtaining several points of the amount of change K in the same way for samples with different amounts of calcium carbonate, plotting the amount of calcium carbonate along the horizontal axis and the amount change K along the vertical axis, and determining the inclination (the amount of change β of the resulting line in the chart. The amount of carbon dioxide originating from carbonate ester can be calculated by applying the amount of change β, the amount of PC, and the amount of calcium carbonate to the following formula.

```
Amount of carbon dioxide of carbonate ester (wt%) =

β × amount of PC (wt%) ×

amount of calcium carbonate (wt%)
```

Since the rate of acid decomposition of PC in the standard samples is small, the total carbon amount measured by acid treatment (Treatment 4) $[Y \times C \times 10^{-3}$ mg/10g] is assumed to be the amount originating from decomposition of calcium carbonate. Based on this assumption, the amount (wt%) of calcium carbonate is calculated from the total carbon amount according to the following formula.

```
Amount of calcium carbonate (wt%) =

(Y × C × 10⁻⁶/10) × 100 × M_CaCO3/Mc          (8)
```

wherein $M_{CaCO3}$ is the molecular weight of calcium carbonate (100) and Mc is the elemental weight of carbon (12). Then, the amount of carbon dioxide originating from carbonate ester can be calculated as follows.

```
Amount of carbon dioxide originating from carbonate ester (wt%)

= (M_CaCO3/Mc) × 10⁻⁵ × PC × β × Y × C
```

(c) Calculation of mass percent of carbon in sample excluding carbonate salt

The mass percent of carbon dioxide generated from 1 g of a sample by the acid treatment in Treatment 4 is $(M_{CO2}/Mc) \times 10^{-5} \times Y \times C$ (wt%). Based on (b) above, the amount of carbon dioxide originating from carbonate ester is $(M_{CaCO3}/Mc) \times 10^{-5} \times PC \times β \times Y \times C$ (wt%). Therefore, the amount of carbon dioxide originating from carbonate salt in the carbon dioxide generated by the acid treatment is $(M_{CO2} - M_{CaCO3} \times PC \times β) \times 10^{-5} \times Y \times C/Mc$ (wt%). Since the mass percent of the total carbon in the sample is $10^{-4} \times X \times A \times$ (wt%) based on Treatment 1, the mass percent of carbon in the sample excluding carbonate salt is shown by the following formula.

```
Mass percent of carbon =

10⁻⁴ × X × A - 10⁻⁵ × (1 - PC × β × (M_CaCO3/M_CO2) × Y × C (wt%)
```

(d) Calculation of $^{14}C$ amount originating from carbonate salt

The carbon dioxide generated from the acid treatment originates from carbonate salt and ester contained in the sample. Since carbonate ester is a material derived from fossil fuel, the amount of $^{14}C$ is 0%. The $^{14}C$ amount originating from carbonate salt is indicated as E%.

Since the $^{14}C$ amount (pMC) of the sample treated with an acid is D%, the amount of $^{14}C$ originating from carbonate salt and the amount of $^{14}C$ in the sample treated with an acid satisfy the following formula.

```
(CO₂ mass percent originating from carbonate salt/

CO₂ mass percent generated during acid treatment) × E = D
```

The following formula is derived from this relationship and the above (c).

```
E (%) = D/{1 - PC × β × (M_CaCO3/M_CO2)}
```

(e) Calculation of $^{14}C$ concentration of portion excluding carbonate salt from sample

Referring to the formula (2), F' which is the $^{14}C$ concentration (pMC: F'%) of the portion excluding carbonate salt from the sample is shown by the formula.

$$F'=\frac{10\times B-D\times\gamma}{10-(1-PC\times\beta\times M_{CaCO3}/M_{CO2})\times\gamma}$$

wherein $\gamma = (Y/X) \times (C/A)$

(f) Calculation of amount (wt%) of biomass carbonate salt in sample

For example, eggshell and seashell contain calcium carbonate mainly made of cyclic carbon (biomass carbonate salt). For example, when eggshell (pMC = G%) is biomass carbonate salt in the sample, the following formula is satisfied.

$$E=\frac{Carbon\ amount\ of\ biomass\ carbonate\ salt\times G}{(1-PC\times\beta\times M_{CaCO3}/M_{CO2})\times10^{-5}\times Y\times C}$$

Based on this formula, the amount of biomass carbonate salt (eggshell) (wt%) can be determined by calculating the amount of carbon of biomass carbonate salt using this formula and dividing the resulting amount by the carbon content of the carbonate salt ($Mc/M_{CaCO3}$).

(g) Calculation of amount (wt%) of organic biomass component in sample

From the $^{14}C$ concentration (pMC: F'%) of the portion excluding carbonate salt from the sample, the cyclic carbon content (%) of this portion can be calculated by the following formula.

```
Cyclic carbon content H' (%) = (F' × 100) /110
```

The mass percent I' (wt%) of carbon originating from cyclic carbon (biomass component) can be calculated by the following formula.

$$I' \ (wt\%) \ = \ (X \ \times \ A \ \times \ 10^{-6} \ - \ Y \ \times \ C \ \times \ 10^{-7}) \ \times \ H'$$

When the biomass component contained in the sample is polylactic acid, since the carbon content according to elementary analysis is 50 wt%, the mass percent of the polylactic acid contained in the sample is as follows.

$$\text{Mass percent of polylactic acid (wt\%) = I'/0.5}$$

The carbon amount and $^{14}$C concentration of materials in a sample calculated by (a) to (g) above are shown in Table 1 and the mass percent (wt%) in the sample is shown in Table 2.
Table 1

| | Total carbon (wt%) | Amount in sample (wt%) | $^{14}$C concentration (pMC:%) |
|---|---|---|---|
| Measured by combustion treatment (entire sample) | $X \cdot A \cdot 10^{-4}$ $\cdots (1)$ | - | B |
| Measured by acid treatment | $Y \cdot C \cdot 10^{-5}$ $\cdots (2)$ | - | D |
| Portion of carbonate salt | $10^{-5} \times Y \times C \times (1 - PC \times \beta \times M_{CaCO3}/M_{CO2})$ $\cdots (3)$ | $(M_{CaCO3}/Mc) \times (3)$ (as $CaCO_3$) | $E = D/(1 - PC \times \beta \times M_{CaCO3}/M_{CO2})$ |
| Portion of carbonate ester | $0.7557^{*} \times PC$ $\cdots (4)$ | PC (as polycarbonate) | O |
| Organic substance other than carbonate ester | (1)-(3)-(4) | Refer to Table 2 | $F' = \dfrac{10 \times B - D \times \gamma}{10 - (1 - PC \times \beta \times M_{CaCO3}/M_{CO2}) \times \gamma}$ |
| Inorganic substance other than carbonate salt | 0 | 100-(sum of upper values) | - |

$M_{CaCO3} = 100$: $M_{CO2} = 44$: $Mc = 12$: $\gamma = (Y/X) \times (C/A)$

PC (wt%) = NMR integrated value $\times$ Internal standard /$\alpha$:

$\alpha$ = integrated value of PC/[PC wt%/Internal standard wt%] * 0.7557 is the carbon content (wt/wt) of polycarbonate.

EP 1 927 857 A1

Table 2

| Sample composition | Subdivision | Assumption | Mass percent in sample (wt%) |
|---|---|---|---|
| Organic component | (1) Biomass component | Carbon content of material x | Cycle carbon content /x |
| | (2) Carbonate ester (non-biomass component) | As polycarbonate | PC (measured from NMR integrated value) |
| | (3) Other non-biomass component | pMC = 0 | - |
| Carbonate salt | (4) Biomass carbonate salt | $CaCO_3$ : pMC = G e.g eggshell | $(M_{CaCO3}/M_C) \times 10^{-5} \times Y \times C \times D/G$ |
| | (5) Non-biomass carbonate salt | $CaCO_3$ e.g limestone | $(M_{CaCO3}/M_C) \times 10^{-5} \times Y \times C \times (1-PC \times \beta \times M_{CaCO3}/M_{CO2} - D/G)$ |
| Inorganic component other than carbonate salt | - | - | - |

PC (wt%) = NMR integrated value $\times$ internal standard/$\alpha$:

$\alpha$ = integrated value of PC/[PC wt%/internal standard wt%] Biomass degree = (1) + (4) (wt%)

EP 1 927 857 A1

As shown in Table 2, according to the measuring method of the invention, the biomass degree can be determined by subdividing the components in a sample to five types. Since the method takes inorganic fillers contained in a sample into account, the biomass degree of a sample can be more accurately determined.

Specific examples of biomass degrees determined by the above-described measurement and calculation are given below. Sample 1 is a chalk containing about 60% of eggshell (mass percent in the sample, hereinafter the same) and cellulose, and sample 2 is a teacup made from a resin which contains about 40% of eggshell and about 10% of cone pole (carbohydrate). The results are shown in Table 3.

In the calculation, the $^{14}C$ concentration (pMC) of eggshell was taken to be 104.66%, and carbohydrate ($C_nH_{2n}O_n$) and cellulose were taken to have a carbon content of 44.07 wt% and a $^{14}C$ concentration (pMC) of 110%.

Table 3

| | | Sample 1 | Sample 2 |
|---|---|---|---|
| Measurement by burning treatment | Amount of sample (g) | 1.0000 | 1.0000 |
| | Trapped mass X (g) after $CO_2$ absorption | 1012.5 | 998.2 |
| | Total carbon concentration A (ppm) | 2.29 | 1.19 |
| | Carbon content of sample (wt%) | 23.20 | 11. 90 |
| | $^{14}C$ concentration pMC: B (%) | 50.50 | 66.00 |
| | Ash | Found | Found |
| Measurement by acid treatment | Amount of sample (g) | 10.0000 | 10.0000 |
| | Trapped mass Y (g) after $CO_2$ absorption | 998.7 | 1002.49 |
| | Total carbon concentration C (ppm) | 4.72 | 10.7 |
| | Carbon content of sample (wt%) | 4.715 | 10.773 |
| | $^{14}C$ concentration pMC:D (%) | 104.66 | 64.26 |
| Existence of carbonate ester | | None | None |
| Results of calculation using measured data | Carbon content of carbonate salt in sample (wt%) | 4.72 | 10.773 |
| | $^{14}C$ concentration pMC:E (%) originating from carbonate salt | 104.66 | 64.26 |
| | Ratio of biomass carbonate salt in sample (wt%) (1) | 39.295 | 55.119 |
| | Carbon content of portion excluding carbonate salt (organic substance) in sample (wt%) | 18.5 | 1.1 |
| | $^{14}C$ concentration pMC:F (%) of portion excluding carbonate salt | 36.68 | 82.59 |
| | Carbon content of organic biomass component (wt%) | 6.16 | 0.85 |
| | Mass percent of organic biomass component (wt%) (2) | 13.99 | 1.92 |
| Biomass degree of sample (content of biomass components ((1)+(2)) wt%) | | 53.28 | 57.04 |

INDUSTRIAL APPLICABILITY

According to the method of the invention, the cyclic carbon content of an entire sample can be more accurately determined, even if the sample contains substances with different cyclic carbon contents. In addition, since the method can measure the mass percent (wt%) of a biomass component in a sample, whether or not the sample is a material with a small environmental load can be easily judged. Therefore, the method can be used for authorization of a material

## EP 1 927 857 A1

(biomass material) with a small environmental load.

**Claims**

1. A method for determining a cyclic carbon content in a sample comprising:

   burning a sample to generate carbon dioxide gas and measuring a total $^{14}$C amount in the sample from the carbon dioxide gas;
   burning the sample to confirm presence or absence of ash;
   when ash is present, treating the sample with an acid to generate carbon dioxide gas and measuring a $^{14}$C amount from the carbon dioxide gas;
   correcting the total $^{14}$C amount in the sample with the $^{14}$C amount measured by the acid treatment; and
   calculating a cyclic carbon content in the sample from the corrected total $^{14}$C amount in the sample.

2. The method according to claim 1, wherein the $^{14}$C amount measured by the acid treatment is taken as a $^{14}$C amount originating from carbonate salt contained in the sample; and
   a cyclic carbon content of the portion excluding the carbonate salt of the sample and/or a cyclic carbon content of the carbonate salt portion are/is calculated.

3. The method according to claim 1, wherein presence or absence of carbonate ester in the sample is measured;
   when carbonate ester is present, the $^{14}$C amount measured by the acid treatment is taken as a $^{14}$C amount originating from carbonate salt and carbonate ester;
   a $^{14}$C amount originating from the carbonate salt is calculated from the $^{14}$C amount measured by the acid treatment; and
   a cyclic carbon content of the portion excluding the carbonate salt of the sample and/or a cyclic carbon content of the carbonate salt portion are/is calculated.

4. The method according to claim 3, wherein the presence or absence of carbonate ester is confirmed by $^{13}$C NMR analysis and IR absorption analysis.

5. The method according to claim 3 or 4, wherein an amount of the carbonate ester is determined by an integrated value obtained by $^{13}$C NMR analysis.

6. The method according to claim 5, wherein an amount of carbon dioxide gas originating from the carbonate ester in the carbon dioxide gas generated by the acid treatment is calculated, based on the amount of the carbonate ester in the sample; and
   the cyclic carbon content of the carbonate salt portion is determined by subtracting the amount of carbon dioxide gas originating from the carbonate ester from the amount of carbon dioxide gas generated by the acid treatment.

7. The method according to any one of claims 1 to 6, wherein the cyclic carbon content in the sample is determined by calculating a cyclic carbon content of the portion excluding carbonate salt of the sample and a cyclic carbon content of the carbonate salt portion, and adding the resultant cyclic carbon contents.

8. A method for determining a content of a cyclic carbon material in a sample comprising:

   determining a cyclic carbon content in a sample according to the method recited in any one of claims 1 to 7; and
   calculating a content (mass%) of a cyclic carbon material from the determined cyclic carbon content in the sample, a total carbon amount of the sample, and a carbon content of each component contained in the sample.

18

FIG. 1

STEP A

```
┌─────────────────────────────────────┐
│ MEASUREMENT OF TOTAL $^{14}$C        │
│ CONCENTRATION (pMC: B%) OF           │
│ SAMPLE BY COMBUSTION TREATMENT       │
└─────────────────────────────────────┘
```

STEP B

CONFIRMATION OF PRESENCE OR ABSENCE OF ASH IN SAMPLE

ABSENT

```
┌─────────────────────────────────────┐
│ CYCLIC CARBON CONTENT (%)            │
│ OF SAMPLE = (B/110) × 100            │
└─────────────────────────────────────┘
```

STEP C    PRESENT

```
┌─────────────────────────────────────┐
│ MEASUREMENT OF $^{14}$C              │
│ CONCENTRATION (pMC: D%) OF           │
│ SAMPLE BY ACID TREATMENT             │
└─────────────────────────────────────┘
```

STEP D

CONFIRMATION OF PRESENCE OR ABSENCE OF CARBONATE ESTER IN SAMPLE

CORRECTION(1)

ABSENT

```
┌──────────────────────┐  ┌──────────────────────┐  ┌──────────────────────┐
│ ASSUME D AS $^{14}$C │  │ CALCULATION OF $^{14}$C │  │ CYCLIC CARBON CONTENT │
│ CONCENTRATION        │  │ CONCENTRATION (pMC: F%) │  │ (%) OF SAMPLE EXCLUDING │
│ (pMC: E%) OF         │  │ OF SAMPLE EXCLUDING  │  │ CARBONATE SALT =      │
│ CARBONATE SALT       │  │ CARBONATE SALT       │  │ (F/110) × 100         │
└──────────────────────┘  └──────────────────────┘  └──────────────────────┘
```

PRESENT
CORRECTION(2)

```
┌──────────────────────┐  ┌──────────────────────┐  ┌──────────────────────┐  ┌──────────────────────┐
│ ASSUME D AS $^{14}$C │  │ CALCULATION OF       │  │ CALCULATION OF $^{14}$C │  │ CYCLIC CARBON CONTENT │
│ CONCENTRATION (pMC:  │  │ $^{14}$C CONCENTRATION │  │ CONCENTRATION (pMC: F'%) │  │ (%) OF SAMPLE EXCLUDING │
│ H%) OF CARBONATE SALT│  │ (pMC: E'%) OF        │  │ OF SAMPLE EXCLUDING  │  │ CARBONATE SALT =      │
│ AND CARBONATE ESTER  │  │ CARBONATE SALT       │  │ CARBONATE SALT       │  │ (F'/110) × 100        │
└──────────────────────┘  └──────────────────────┘  └──────────────────────┘  └──────────────────────┘
```

FIG. 2

AIR

EXHAUST GAS

**INTERNATIONAL SEARCH REPORT**

| | International application No. |
|---|---|
| | PCT/JP2006/317082 |

A. CLASSIFICATION OF SUBJECT MATTER
*G01N33/00*(2006.01)i, *G01N33/22*(2006.01)i, *G01G01N24/08*(2006.01)i, *G01N21/35*(2006.01)i, *G01N27/62*(2006.01)i

According to International Patent Classification (IPC) or to both national classification and IPC

B. FIELDS SEARCHED

Minimum documentation searched (classification system followed by classification symbols)
G01N33/00-33/46, G01N31/00-31/22, G01N24/00-24/14, G01N21/35, G01R33/00-33/20

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2005-17018 A  (Mitsubishi Heavy Industries, Ltd.), 20 January, 2005 (20.01.05), Full text; all drawings (Family: none) | 1-8 |
| A | JP 2003-185634 A  (Idemitsu Petrochemical Co., Ltd.), 03 July, 2003 (03.07.03), Full text; all drawings (Family: none) | 1-8 |
| A | JP 2004-198239 A  (Idemitsu Petrochemical Co., Ltd.), 15 July, 2004 (15.07.04), Full text; all drawings (Family: none) | 1-8 |

☐ Further documents are listed in the continuation of Box C.   ☐ See patent family annex.

| | |
|---|---|
| *  Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| 21 November, 2006 (21.11.06) | 28 November, 2006 (28.11.06) |

| Name and mailing address of the ISA/<br>Japanese Patent Office | Authorized officer |
|---|---|
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003185634 A

- JP 2004198239 A